# EUROPÄISCHE PATENTANMELDUNG

(11) **EP 1 674 563 A1**
(43) Veröffentlichungstag der Anmeldung: **28.06.2006**
(21) Anmeldenummer: 05028411.6
(22) Anmeldetag: 23.12.2005
(51) Int. Cl.: C12N 5/06, A61B 17/435, A61B 18/20

(54) **Embryonenerhaltende Gewinnung pluripotenter embryonaler Stammzellen, derart gewonnene Stammzellen und Verwendung derselben**

(30) Priorität: 23.12.2004 DE 102004062184
(71) Anmelder: Würfel, Wolfgang, 82234 Oberpfaffenhofen (DE)
(72) Erfinder: Würfel, Wolfgang, 82234 Oberpfaffenhofen (DE)
(74) Vertreter: von Samson-Himmelstjerna, Friedrich

(57) **Zusammenfassung**

Verfahren zur embryonenerhaltenden Gewinnung pluripotenter embryonaler Stammzellen, bei dem
a) in der Zona pellucida von fixierten Blastocysten ein Kanal geöffnet wird,
b) durch diesen Kanal ein geeignetes Instrument zur Mobilisierung von Zellen durch den Trophectoderm bis zur inneren Zellmasse geführt wird und mit diesem Instrument einige Stammzellen der inneren Zellmasse mobilisiert werden, und
c) die mobilisierten Stammzellen abgesaugt werden;
durch obiges Verfahren erhältliche Stammzellen sowie Verwendung derselben zur Behandlung einer Verletzung oder einer Krankheit.

## Beschreibung

Stammzellen unterscheiden sich von normalen differenzierten Zellen prinzipiell durch ihr Vermögen zur unbegrenzten Teilung und das Potential zur Entwicklung in ganz verschiedene Zellarten mit charakteristischen Phänotypen und spezialisierten Funktionen. Dieses Potential macht sie sehr interessant für vielfältige Anwendungen in der Pharmazie und Medizin. Insbesondere der Ersatz und/oder die Regeneration von zerstörtem oder unzureichend funktionierendem Gewebe eines Patienten ist hierbei ein bevorzugtes Ziel.

Als Quelle solcher Stammzellen kommen grundsätzlich zwei Möglichkeiten in Frage. Die erste Möglichkeit ist die Gewinnung von Stammzellen aus Präimplantationsembryonen. Bei sämtlichen etablierten Verfahren kommen dabei jedoch Methoden zum Einsatz, die zur Zerstörung der Embryonen führen (siehe z.B. WO 01/29206 und die dort zitierte Literatur). Für den medizinisch besonders interessanten Fall der Gewinnung menschlicher Stammzellen ist diese Praxis daher aus ethischen Gründen umstritten und wird vielfach strikt abgelehnt.

Eine Alternative ist die Gewinnung sogenannter "adulter" Stammzellen aus bereits differenziertem Körpergewebe. Diese Stammzellen sind jedoch nach bisheriger Erfahrung den embryonalen Stammzellen hinsichtlich ihres Differenzierungspotentials unterlegen. Im Gegensatz zu den pluripotenten embryonalen Stammzellen, die sich in Zelltypen aller drei Keimblätter differenzieren können, sind die adulten Stammzellen in aller Regel höchstens totipotent, d.h. sie können sich nur in verschiedene Zellen eines Keimblatt-Typs entwickeln. Einzelne Forschungsergebnisse in jüngster Zeit sprechen zwar auch bestimmten adulten Stammzellen eine Pluripotenz zu. Diese Ergebnisse wurden jedoch noch nicht bestätigt bzw. wieder angezweifelt.

Vor diesem Hintergrund zielt die vorliegende Erfindung auf die Bereitstellung eines neuen Verfahrens zur Gewinnung pluripotenter embryonaler Stammzellen, der so gewonnenen Stammzellen selbst und deren Verwendung ab.

Erfindungsgemäß wird dieses Ziel mit den Gegenständen der Ansprüche 1, 9 und 10 erreicht; insbesondere also mit
einem Verfahren zur embryonenerhaltenden Gewinnung embryonaler Stammzellen, bei dem
- a): in der Zona pellucida von fixierten Blastocysten ein Kanal geöffnet wird,
- b): durch diesen Kanal ein geeignetes Instrument zur Mobilisierung von Zellen durch das Trophectoderm bis zur inneren Zellmasse geführt wird und mit diesem Instrument einige Stammzellen der inneren Zellmasse mobilisiert werden, und
- c): die mobilisierten Stammzellen abgesaugt werden;
Stammzellen, die nach dem erfindungsgemäßen Verfahren gewonnen wurden,
sowie
einer Verwendung der erfindungsgemäßen Stammzellen zur Behandlung einer Verletzung und/oder einer Krankheit.

Das erfindungsgemäße Verfahren hat den großen Vorteil, daß mit ihm pluripotente embryonale Stammzellen ohne die ethisch bedenkliche Vernichtung von Embryonen erhältlich sind.

Weitere Aspekte der vorliegenden Erfindung betreffen die Verwendung der erfindungsgemäß erhaltenen Stammzellen zur therapeutischen Behandlung von Säugern, insbesondere Menschen.

Die vorliegende Erfindung beruht auf der überraschenden Erkenntnis, dass eine oder mehrere Stammzellen aus der inneren Zellmasse von Embryos im Blastocysten-Stadium isoliert werden können, ohne die Lebensfähigkeit der Embryos zu beeinträchtigen. Blastocysten sind Embryonen in einem Alter von etwa 4-7 Tagen und zeigen im Gegensatz zu jüngeren Embryonen bereits eine Differenzierung in die innere Zellmasse, aus der sich später der Fötus entwickelt, und in dem Trophectoderm, aus dem sich extra-embryonische Gewebe entwickeln.

Die Blastocysten können von einem beliebigen Vertebraten, vorzugsweise einem Säuger, stammen. Besonders bevorzugt stammen sie von einem domestizierten Tier, z.B. Pferd, Rind, Schaf, Schwein, oder einem Primaten, z.B. dem Menschen.

Das erfindungsgemäße Verfahren unterscheidet sich von der aus der Präimplantationsdiagnostik (PID) bereits bekannten Blastocystenbiopsie grundsätzlich darin, dass bei der PID Zellen des Trophectoderms und nicht der inneren Zellmasse entnommen werden.

Das erfindungsgemäß angewandte Verfahren umfasst drei Hauptschritte:
- a): Öffnung eines Kanals in der Zona pellucida (der äußeren Schutzhülle) von Blastocysten;

- b): Einführung eines Instruments zur Mobilisierung von Zellen durch diesen Kanal und durch den Trophectoderm und Mobilisierung einiger Stammzellen der inneren Zellmasse mit diesem Instrument; und
- c): Absaugen der mobilisierten Stammzellen.

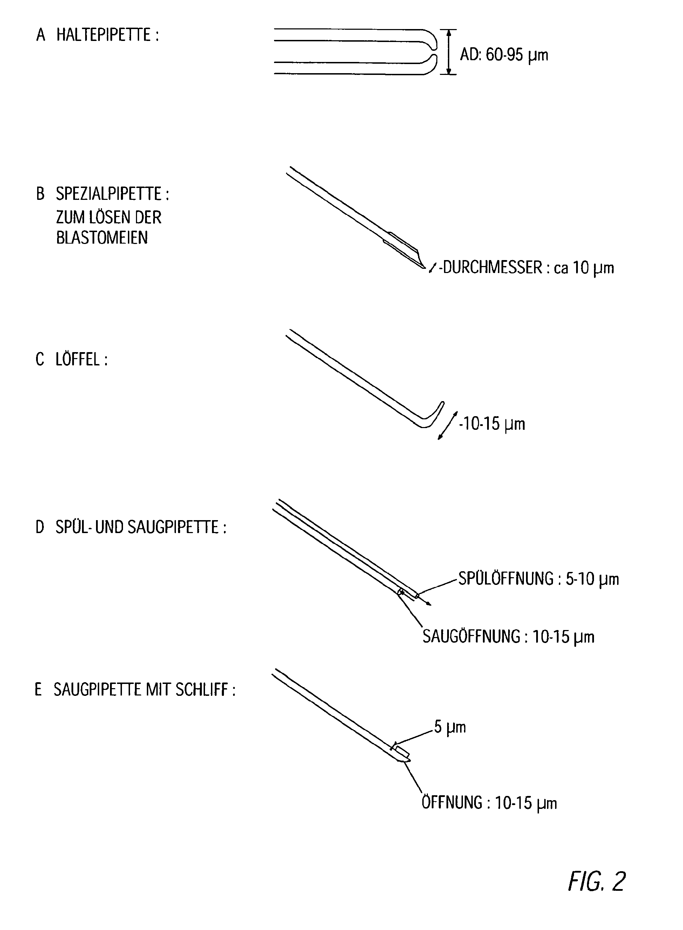

Vor der Öffnung der Zona pellucida muß die Blastocyste zunächst fixiert werden. Dies kann beispielsweise mit einer Haltepipette geschehen. Die Haltepipette wird typischerweise einen Außendurchmesser von etwa 60-95 µm aufweisen (vgl. Fig. 2A).

Zur gefahrlosen Perforation der Zona pellucida sind aus der Implantationsmedizin bereits verschiedene Verfahren bekannt. Möglich sind chemische Techniken mit z.B. saurer Tyrode-Lösung, mechanische Techniken und, erfindungsmäß besonders bevorzugt, Laser-Techniken (vgl. Human Reproduction Bd. 15, S. 65-67, 2000).

Eine typische Vorgehensweise bei Verwendung eines Lasers besteht darin, dass der Laser durch mehrere kurze Impulse von jeweils zwischen 10 und 20 ms eine Öffnung von etwa 20 µm Durchmesser erzeugt.

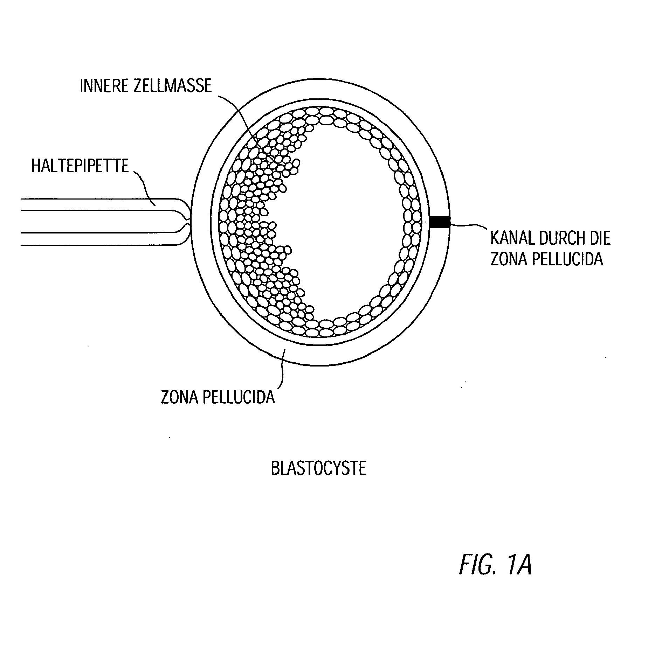

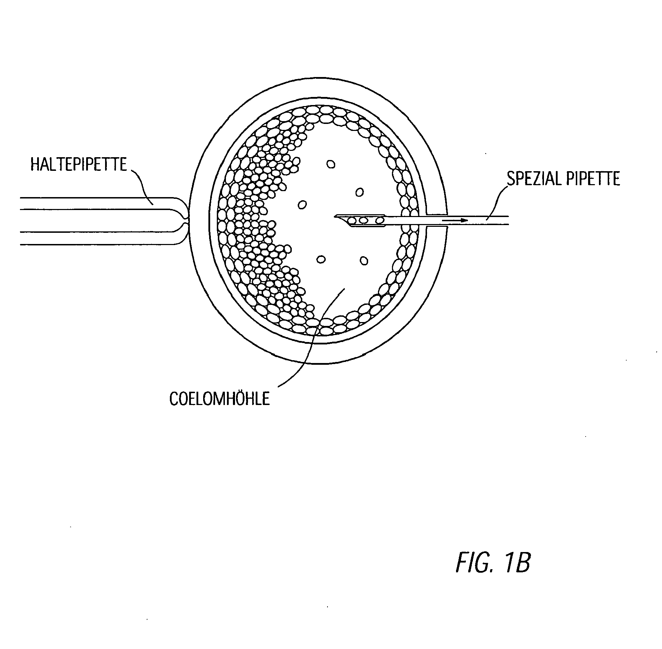

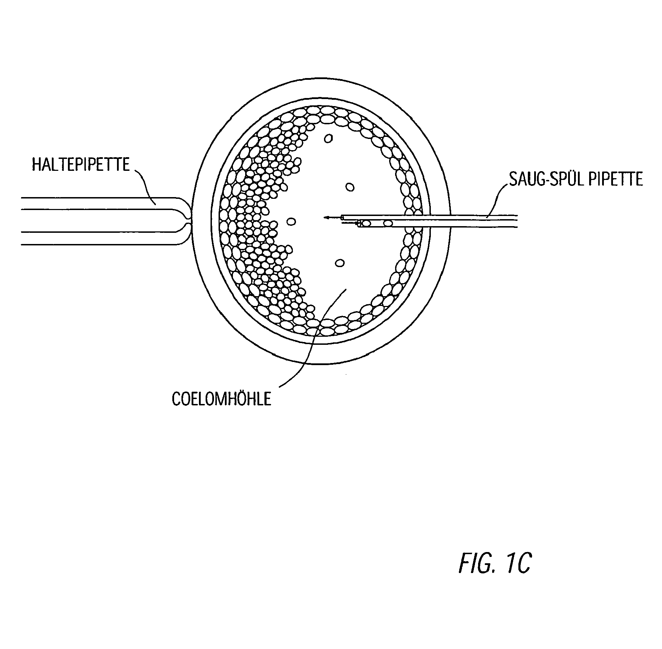

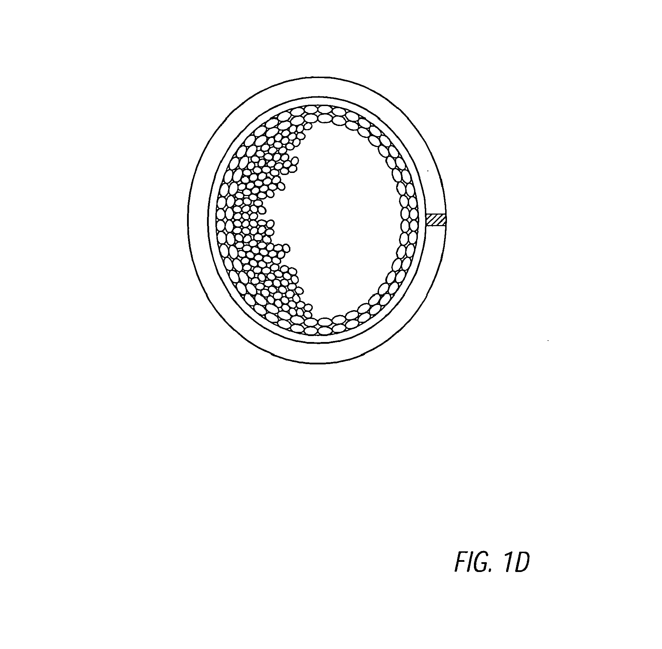

In diese Öffnung wird dann das Instrument zur Mobilisierung der Stammzellen eingeführt und durch die anschließende Schicht des Trophectoderms bis zur inneren Zellmasse geführt (siehe Fig. 1). Ein geeignetes Mobilisierungsinstrument ist beispielsweise eine Spezialpipette, die an mindestens zwei Seiten einen messerartigen Aufsatz aufweist, Durchmesser ca. 10 µm (siehe Fig. 2B). Durch vorsichtiges Drehen der Pipette können einzelne Zellen der inneren Zellmasse mobilisiert werden. Eine Alternative ist die Verwendung eines kleinen mikrochirurgischen "Löffels", Durchmesser ca. 10-15 µm (Fig. 2C).

Anschließend werden die mobilisierten Zellen mit Hilfe einer Saugpipette oder mit einer Saug-Spül-Pipette (Fig. 2D) entnommen. Falls die Saugpipette selbst einen messerartigen Aufsatz aufweist (Fig. 2E), kann sie auch als Mobilisierungsinstrument dienen. Allerdings ist die Verwendung der oben beschriebenen Spezial pipette effizienter. Darüber hinaus ist die Verwendung einer Saug-Spül-Pipette bevorzugt, da sie es ermöglicht, den beim Absaugen der Zellen erzeugten Unterdruck im Blastocysteninnenraum zu neutralisieren, und somit eine etwaige Schädigung des Blastocysten-Embryos durch den Unterdruck zu vermeiden. Eine geeignete Saug-Spül-Pipette hat eine Spülöffnung von etwa 5-10 µm und eine Saugöffnung von etwa 10-15 µm.
Eine geeignete Saugpipette hat ebenfalls eine Öffnung von etwa 10-15 µm.

Nach der Entnahme werden die Zellen in geeignetes Kulturmedium überführt und nach bekannten Verfahren zur Züchtung embryonaler Stammzellen (siehe z.B. WO 96/22362 und WO 01/29206) kultiviert. Die Züchtung beinhaltet typischerweise die Kultivierung auf Maus-Fibroblasten-Feederzellschichten. Statt Maus-Fibroblasten können jedoch auch Feederzellen anderen Ursprungs, z.B. Primatenzellen, verwendet werden. In einer Ausführungsform beinhaltet das Kultivierungsverfahren das Ausplattieren der gewonnenen Zellen auf einer Maus-Fibroblastenschicht, wobei Zellansammlungen gebildet werden, das Entfernen der Zellansammlungen und das Dissoziieren der Zellansammlungen zu dissoziierten Zellen und erneutes Ausplattieren auf Fibroblasten-Feederzellschichten. Ein bevorzugtes Medium für die Kultivierung embryonaler Stammzellen ist das in WO 96/22362 beschriebene "ES"-Medium, welches aus 80% DMEM (Dulbeccos Modified Eagle's Medium), 20% fötalem Kalbserum (FBS), 0,1 mM β-Mercaptoethanol und gegebenenfalls kleinen Mengen weiterer Additive, z.B. Aminosäuren, Antibiotika etc., besteht. Es können jedoch auch andere im Stand der Technik bekannte geeignete Medien eingesetzt werden.

Die erfindungsgemäß erhaltenen Stammzellen können in der Pharmazie und Medizin bzw. Veterinärmedizin, z.B. zur Behandlung einer Verletzung oder Krankheit, eingesetzt werden. Eine bevorzugte Anwendung ist der Ersatz oder die Regeneration eines geschädigten oder fehlenden Gewebes oder Organs bei einem menschlichen oder tierischen Patienten. Die Behandlung kann beispielsweise dadurch erfolgen, dass eine therapeutisch wirksame Menge der Stammzellen in den Körper des Patienten eingebracht wird.

### FIGURENBESCHREIBUNG

- **Fig. 1**: zeigt schematisch die Hauptschritte bei der Durchführung des erfindungsgemäßen Verfahrens, insbesondere zeigt
- Fig. 1A: einen Kanal durch die Zona pellucida eines Blastocysten
- Fig. 1B: das Mobilisieren von Zellen der inneren Zellmasse mit einer Spezialpipette
- Fig. 1C: das Absaugen der mobilisierten Zellen mit einer Saug-Spül-Pipette
- Fig 1 D: den Blastocysten nach Abschluß des Verfahrens und selbstständiger Schließung des Kanals.
- Fig. 2: zeigt verschiedene spezielle Werkzeuge zur Durchführung des erfindungsgemäßen Verfahrens, insbesondere zeigt
- Fig. 2A: eine Haltepipette
- **Fig. 2B**: eine Spezialpipette zur Mobilisierung von Zellen
- **Fig. 2C**: einen mikrochirurgischen "Löffel"
- Fig. 2D: eine Saug-Spül-Pipette
- Fig. 2E: eine Saugpipette mit Messeransatz.

Das folgende Beispiel dient der weiteren Erläuterung der Erfindung, ohne diese jedoch darauf zu beschränken.

### BEISPIEL

Ein humaner Embryo im Blastocysten-Stadium (5 Tage alt) wird mit einer Haltepipette (ICSI-Haltepipette mit Winkel; Außendurchmesser 60-95 µm (Humagen)) fixiert. Dann wird durch mehrere kurze Impulse von jeweils zwischen 10 und 20 ms Dauer mit einem Laser Modell Fertilase MTM (Medical technologies Montreux S.A. (1,48 µm) eine Öffnung von etwa 20 µm Durchmesser in der Zona pellucida des Blastocysten erzeugt.

In diese Öffnung wird dann eine Spezialpipette eingeführt und durch die anschließende Schicht des Trophectoderms bis zur inneren Zellmasse geführt. Durch vorsichtiges Drehen der Pipette werden einzelne Zellen der inneren Zellmasse mobilisiert. Die Spezialpipette hat einen Durchmesser von ca. 10 µm und weist an zwei Seiten einen messerartigen Aufsatz auf.

Anschließend werden die mobilisierten Zellen mit Hilfe einer Saug-Spül-Pipette, (Spülöffnung etwa 5-10 µm und Saugöffnung etwa 10-15 µm) entnommen.

Nach der Entnahme schließt sich der Entnahmekanal im Blastocysten von selbst. Die entnommenen Zellen werden in Kulturmedium auf DMEM-Medium überführt und nach bekannten Verfahren zur Züchtung embryonaler Stammzellen kultiviert.

## Patentansprüche

1. Verfahren zur Gewinnung pluripotenter embryonaler Stammzellen, bei dem
a) in der Zona pellucida von fixierten Blastocysten ein Kanal geöffnet wird,
b) durch diesen Kanal ein geeignetes Instrument zur Mobilisierung von Zellen durch den Trophectoderm bis zur inneren Zellmasse geführt wird und mit diesem Instrument einige Stammzellen der inneren Zellmasse mobilisiert werden, und
c) die mobilisierten Stammzellen abgesaugt werden.

2. Verfahren nach Anspruch 1, **dadurch gekennzeichnet, dass** die Fixierung der Blastocysten mittels einer Haltepipette erfolgt.

3. Verfahren nach Anspruch 1 oder 2, **dadurch gekennzeichnet, dass** die Öffnung der Zona pellucida mittels eines Lasers erfolgt.

4. Verfahren nach einem der vorstehenen Ansprüche, **dadurch gekennzeichnet, dass** das Instrument zur Mobilisierung der Stammzellen eine Spezialpipette mit mindestens einem beidseitigen Messer oder ein mikrochirurgischer "Löffel" ist.

5. Verfahren nach einem der vorstehenden Ansprüche, **dadurch gekennzeichnet, dass** die mobilisierten Stammzellen mit einer Saug-Spül-Pipette abgesaugt werden.

6. Verfahren nach einem der vorstehenden Ansprüche, **dadurch gekennzeichnet, dass** die Stammzellen nach ihrer Entnahme in einem Kulturmedium für embryonale Stammzellen kultiviert und vermehrt werden.

7. Verfahren nach einem der vorstehenden Ansprüche, **dadurch gekennzeichnet**, das die Blastocysten von einem Vertebraten, vorzugsweise einem Säuger, stammen.

8. Verfahren nach Anspruch 7, **dadurch gekennzeichnet, dass** die Blastocysten von einem Primaten, z. B. einem Menschen, stammen.

9. Stammzellen, erhältlich mit dem Verfahren nach einem der Ansprüche 1 bis 8.

10. Verwendung der Stammzellen nach Anspruch 9 zur Behandlung einer Verletzung oder einer Krankheit.

11. Verwendung nach Anspruch 10, **dadurch gekennzeichnet, dass** die Behandlung in der Regeneration oder dem Ersatz eines geschädigten oder fehlenden Gewebes oder Organs besteht.

12. Verwendung nach Anspruch 10 oder 11, **dadurch gekennzeichnet, dass** eine therapeutisch wirksame Menge der Stammzellen in den Körper eines tierischen oder menschlichen Patienten eingebracht wird.
